Europäisches Patentamt

**European Patent Office**

**Office européen des brevets**

(19)

(11) Publication number: **0 176 246**
**A2**

(12) # EUROPEAN PATENT APPLICATION

(21) Application number: **85306021.8**

(22) Date of filing: **23.08.85**

(51) Int. Cl.⁴: **C 12 Q 1/38**
**C 12 Q 1/00**

(30) Priority: **27.08.84 US 644746**

(43) Date of publication of application:
**02.04.86 Bulletin 86/14**

(84) Designated Contracting States:
**AT CH DE FR GB IT LI NL SE**

(71) Applicant: **JOHNSON & JOHNSON DENTAL PRODUCTS COMPANY**
**20 Lake Drive CN 7060**
**East Windsor New Jersey 08520(US)**

(72) Inventor: **Zahradnik, Robert Thomas**
**1310 Trapelo Road**
**Waltham, MA 02145(US)**

(72) Inventor: **Dankers, Ingrid Ieva**
**266 Forest Street**
**Arlington, MA 02147(US)**

(74) Representative: **Jones, Alan John et al,**
**CARPMAELS & RANSFORD 43 Bloomsbury Square**
**London, WC1A 2RA(GB)**

(54) **Method and kit for detecting the presence of gum disease.**

(57) A method and kit for the detection of proteolytic enzymes in gingival crevicular fluid to aid in diagnosing the presence of inflammatory gum disease. A sample of gingival crevicular fluid is taken up in an absorbent collector, which is then placed in contact with insoluble immobilized collagen containing a bound label such as a dye. After incubation for a short time, if there are any proteolytic enzymes in the sample of fluid, the collagen is degraded to soluble fragments which are taken up in the absorbent sample collector, and are detected by measuring the presence of the label on the collector.

EP 0 176 246 A2

-1-

METHOD AND KIT FOR DETECTING THE PRESENCE OF GUM DISEASE

The invention relates to a method and to a kit for detecting the presence of gum disease by detecting the presence of neutral proteolytic enzymes, especially collagenase.

BACKGROUND OF THE INVENTION

Periodontal disease affects a large proportion (perhaps 75%) of the adult population. It is the principal cause of loss of teeth in adults over 35 years of age. The most effective means for combating periodontal disease is good dental hygiene and early detection. A precursor condition to periodontal disease is simple gum inflammation ("gingivitis"). Gingivitis is accompanied by an increase in the flow of an exudate known as "gingival crevicular fluid" ("GCF"). Many studies have indicated that an analysis of the components found in GCF can be of significant utility in detecting the presence of gum disease, even in its early stages before clinically observable inflammation occurs. For instance, see Golub et al., GINGIVAL CREVICULAR FLUID: A NEW DIAGNOSTIC AID IN MANAGING THE PERIODONTAL PATIENT, Oral Science Reviews 8, 49 - 61 (1976). It has been found that GCF contains neutral proteolytic enzymes, particularly collagenase, and that the concentration of such enzymes in GCF increases substantially when gingivitis is present. For instance, see Golub et al., COLLAGENOLYTIC ACTIVITY OF HUMAN GINGIVAL CREVICE FLUID, J. Dent. Res. 53, No. 6, 1501 (1974), and Golub et al., COLLAGENOLYTIC ACTIVITY OF CREVICULAR FLUID AND OF ADJACENT GINGIVAL TISSUE, J. Dent. Res. 58, No. 11, 2132 (1979).

The major limitation on the development of a practical test for measuring a component of GCF has been the small volume of fluid that can be obtained from a patient in a clinically relevant sampling time of one to three minutes. The fluid volume can range from only 0.1 to 0.5 microliters. Conventional assays are not sensitive enough or require the use of complicated and costly apparatus, long test times, or the use of reagents that require special handling (e.g., radioisotopes).

This invention provides a means for early detection of gum diseases by providing a method and a kit for the detection of neutral proteolytic enzymes, particularly collagenase, in GCF. The invention is easy to use in that simple, inexpensive equipment is used and the procedures are uncomplicated, with no mixing, weighing, pipetting, or separation steps required. The test is sensitive in that it detects the presence of very small quantities of neutral proteolytic enzymes in as little as 0.1 microliter of GCF, and the test is fast so that it can be performed on the patient and the results obtained during one visit to the dentist's office by the patient. The test, although semi-quantitative, will give an excellent estimate of the severity of the patient's condition since collagenase activity has been shown to differ by as much as 10 to 15 times between normal and diseased pockets. Because the test is easy to perform, it is less dependent upon the skill of the operator than are other tests for the detection of gum disease.

The traditional visual examination of soft gingival tissue must be done by a dentist rather than a dental auxilliary. It is difficult to standardize these periodontal indices among clinicians so that the subjective nature of these observations make data interpretation difficult and can result in variable treatment results. Sophisticated instruments have been employed in an attempt to provide an objective measure of the disease. However, the instruments require a certain level of operator familiarity and need frequent calibration and maintenance; for example, the phase-contrast microscope used in the Keyes technique (Keyes, et al., Diagnosis of Crevicul or a Dicular Infections: Disease-Associated Bacterial Patterns in Periodontal Lesions, in Host-Parasite Interactions in Periodontal Diseases (eds., Genco and Mergenhagen), 1982 American Society for Microbiology, Washington, D.C., p. 395.) or the Periotron instrument for use in GCF volume analysis (U.S. Patent No. 3,753,099).

## BRIEF SUMMARY OF THE INVENTION

The method of the invention includes the steps of collecting a sample of gingival crevicular fluid from the patient by using an absorbent sampling device (such as a paper strip), placing the absorbent device containing the sample in operative contact with immobilized insoluble collagen containing a bound label such as a covalently bonded dye, and keeping the device in contact with the immobilized insoluble collagen for a predetermined period of time, preferably at moderately elevated temperature such as body temperature (about 37°C.) If there is any collagenase or other neutral proteolytic enzyme present in the sample, breakdown products of the collagen, containing the label, will be transferred to the absorbent sampling device and can be detected there. (Neutral proteolytic enzymes are enzymes that are optimally active at physiological or neutral pH, and are capable of degrading collagen.)

The invention also provides a kit for practicing the method outlined above. Said kit includes an absorbent GCF sampling device (preferably an absorbent paper strip), and immobilized insoluble collagen containing a bound label

21

such as a covalently bonded dye. Preferably, the kit also includes a means for maintaining the immobilized insoluble collagen at a moderately elevated temperature during the period when it is maintained in contact with the sample.

## THE PRIOR ART

In Kleinberg et al., U.S. Patent No. 3,753,099, there is disclosed a device and method for the quantitative measurement of fluid absorbed in paper strips. The invention is used for the measurement of the volume of absorbed GCF as an aid in the diagnosis of inflammatory gum disease. A commercial device, the "Periotron", marketed by Harco Electronics Limited, Winnipeg, Canada, apparently utilizes the broad principles described in this patent.

Rinderknecht et al., in A NEW ULTRASENSITIVE METHOD FOR THE DETERMINATION OF PROTEOLYTIC ACTIVITY, Clinica Chimica Acta 21, 197-203 (1968), describe a test for detecting the presence of proteolytic enzymes such as collagenase. The test is based on an insoluble substrate derived from hide powder labeled covalently with Remazolbrilliant Blue. The substrate, enzyme sample, and a buffer solution were incubated at 37°C. for 30 minutes, after which time the mixture was filtered. The filtrates were assayed spectrophotometrically, with the amount of color imparted to the filtrate being an indication of the amount of the insoluble hide substrate that had been solubilized.

Preti et al., in U.S. Patent No. 4,334,540, disclose a method for diagnosing periodontal disease by the detection of pyridine compounds.

There are a number of United States patents that disclose methods or assays for measuring or detecting proteolytic enzymes. Illustrative are Sakakibara et al., No. 4,176,009, Richardson et al., No. 4,318,986, Yaron et al., No. 4,314,936, Smith et al., No. 4,294,923, Berger et al., No. 4,278,763, Monget et al., No. 4,277,561, and Pollard, No. 4,260,681.

In the two Golub et al. J. Dent. Res. articles cited above, procedures utilizing insoluble collagen fibrils to detect the presence of collagenase in GCF were described. In the 1979 article, a radioactive labeled $C^{14}$ collagen fibril was used. Following three days incubation with the gingival fluid sample, the substrate is centrifuged and the supernatant, containing solubilized collagen fragments, is collected and assayed using a gamma counter or a radioactive isotope detector. In the 1974 article, insoluble collagen fibrils were suspended in a gel matrix and filter papers containing the GCF samples were placed directly onto the gel and incubated at 37°C. for five days. The gels were then centrifuged and the supernatant and residues were hydrolyzed in 6N HCl and the hydrolyzate was analyzed for hydroxyproline, a degradation product of collagen. Hydroxyproline is found only in collagen and a few plant proteins, and is therefore a good marker for degraded collagen.

## DETAILED DESCRIPTION OF THE INVENTION

The process of the invention uses an absorbent sample collector to collect GCF from the patient. The preferred sample collector is an absorbent strip, such as an absorbent paper strip, which is inserted in the gingival crevice for a predetermined period of time, for instance, for from about 30 to about 90 seconds. The absorbent collector containing the sample of GCF is removed from the gingival crevice and is immediately placed in contact with immobilized insoluble collagen containing a bound label such as a dye. The collagen with the GCF sample in contact therewith is incubated at moderately elevated temperature, e.g., 37°C. for a short period of time, such as 15 minutes, and the collector containing the GCF sample is removed. If the GCF sample contains a neutral proteolytic enzyme such as collagenase, some of the immobilized insoluble collagen that had been in contact with the GCF sample is solubilized and is taken up in the absorbent collector, where the label such as a dye facilitates the detection of the solubilized collagen.

The preferred absorbent GCF sample collector is an absorbent paper strip. Such a strip can be described as follows:

The preferred strip consists of a cellulose filter paper of high purity and low ash (e.g., the Schleicher & Schuell No. 497 or Whatman No. 52). Both papers are hardened with excellent wet strength, and are both about 0.18 mm thick. The size and configuration of the strip is such that it can be easily placed into the sulcus of both anterior and posterior teeth. The preferred strip is rectangular in shape; the width is 2 mm and the length is 14 mm with a vinyl-type support layer on half of the paper to facilitate handling and to prevent bending during placement.

The collagen that is used in the invention is insoluble, that is, it is substantially insoluble in neutral (e.g., pH=6 to 8) aqueous media. The collagen is also immobilized. Preferably, it is immobilized by suspension in an aqueous gel of, e.g., agar or the like. The preferred label for use with the collagen is a covalently bound dye, that is, a dye that will not be extracted by neutral aqueous media. Remazolbrilliant blue is a preferred dye because it binds to collagen through the formation of ether linkages that do not break in neutral aqueous media. Orcein or the azo dyes can also be used. The preparation of the preferred immobilized insoluble labeled collagen is as follows:

| Preferred Formula | % (w/w) |
|---|---|
| Agarose | 1.00 |
| Carboxymethyl cellulose, CMC | 0.80 |
| Dyed Collagen Powder | 4.00 |
| Preservative (e.g., sodium azide) | 0.04 |
| TRIS Buffer, pH 7.6 | 94.16 |
| | 100.00 |

TRIS buffer is a 0.61 per cent (w/w) aqueous solution of tris(hydroxymethyl)aminomethane. Agarose is a purified linear galactan hydrocolloid isolated from agar. It is purer and has better defined gel properties (e.g., gel strength, clarity, and gelling and melting temperatures) than agar.

The dyed collagen powder is prepared by essentially following the procedure described by Rinderknecht in the above-cited 1968 article, except that the dye to collagen ratio and the reaction times have been increased in order to bind additional dye molecules to the collagen backbone. The formula weight of dyed collagen powder, sieved to uniform particle size of approximately 90 to 125 micrometers in diameter, is added to a mix of CMC and the preservative in 2/3 of the formula weight of the buffer. The solution is then warmed to between 40° and 50°C. and a premix of agarose in 1/3 of the formula weight of the buffer is added. The collagen gel can be dispensed into convenient unit-dose containers and refrigerated overnight before use. Development of maximum gel strength is enhanced by cooling below the gelling temperature long enough for the three-dimensional gel network to tighten. Maximum gel strength occurs if the gel is cooled for one

0176246

day at, e.g., 4°C. Because the reaction area under the absorbent sample strip is generally limited to 2 to 8 mm it is important that the dyed collagen powder be properly sized and dispersed uniformly throughout the gel matrix to achieve uniform color development in the sample collector.

The absorbent strip containing the sample of GCF is placed in contact with the immobilized insoluble collagen, and is then maintained at moderately elevated temperature for about 30 minutes. A temperature of 37°C. is satisfactory. A constant temperature oven of conventional design can be used for the incubation. During the incubation, any collagenolytic enzymes present in the GCF will diffuse out into the gel and begin to cleave the dyed collagen fibrils into small, soluble fragments. The gel tends to confine the reaction space to a narrow zone just below the absorbent collector containing the GCF sample. A fraction of the dyed soluble collagen fragments, generated by the enzymatic action of collagenase or other proteolytic enzyme, will diffuse into the sample-containing absorbent collector. The intensity of the dye color in the collector at the completion of the test is related to the original enzyme concentration present in the GCF sample, and the area of the collector that is dyed provides a semi-quantitative measure of the volume of the GCF sample. This is useful information because both the concentration of collagenolytic enzymes in the GCF and the volume of GCF are considered to be indications of the severity of inflammatory gum disease.

CLAIMS

1.    Method for the detection of proteolytic enzyme in gingival crevicular fluid which comprises the steps of:

(a)    placing an absorbent sample collector containing a sample of gingival crevicular fluid in operative contact with immobilized insoluble collagen containing a bound label; and

(b)    maintaining said collector containing said sample in operative contact with said collagen for a period of time and at a temperature sufficient to permit enzymes present in said sample to degrade said collagen to water-soluble fragments, whereby said water-soluble fragments are absorbed in said sample collector and are detected therein.

2.    The method of claim 1 wherein said label is a covalently bound dye.

3.    The method of claim 2 wherein said dye is Remazolbrilliant blue.

4.    The method of any one of claims 1 to 3 wherein said collagen is immobilized in a gel.

5.    The method of any one of claims 1 to 4 wherein said absorbent sample collector is a paper strip.

6.    A kit for the detection of proteolytic enzymes in gingival crevicular fluid which comprises:
(a)    an absorbent device for the collection of gingival crevicular fluid; and
(b)    immobilized insoluble collagen containing a label bound to said collagen.

7.    The kit of claim 6 wherein said absorbent device  is a paper strip.

**0176246**

8. The kit of claim 6 or claim 7 wherein said collagen is immobilized in a gel.

9. The kit of any one of claims 6 to 8 wherein said label is a dye covalently bound to said collagen.

10. The kit of claim 9 wherein said dye is Remazolbrilliant blue.